# EUROPEAN PATENT APPLICATION

(11) **EP 2 295 600 A1**
(43) Date of publication of application: **16.03.2011**
(21) Application number: 09773501.3
(22) Date of filing: 01.07.2009
(51) Int. Cl.: C12Q 1/68, C12N 15/09, G01N 33/53, G01N 37/00

(54) **METHOD AND TEST STRIP FOR DETECTING SENSITIVITY TO ISONIAZID IN MYCOBACTERIUM TUBERCULOSIS**

(30) Priority: 02.07.2008 JP 2008173477
(71) Applicant: NIPRO CORPORATION, Kita-ku Osaka-shi, Osaka 531-8510 (JP); National Center for Global Health and Medicine, Tokyo 162-8655 (JP)
(72) Inventor: KIRIKAE, Teruo, Tokyo 162-8655 (JP); ANDOU, Hiroki, Tokyo 162-8655 (JP); SUETAKE, Toshinori, Osaka-shi Osaka 531-8510 (JP); NAKAMURA, Tomohiko, Osaka-shi Osaka 531-8510 (JP)
(74) Representative: Hiebl, Inge Elisabeth
(86) International application number: PCT/JP2009/062033
(87) International publication number: WO 2010/001923

(57) **Abstract**

According to the present invention, novel means for more reliably detecting sensitivity to Isoniazid (INH) in *Mycobacterium tuberculosis,* that is, detecting whether or not *M. tuberculosis* is an INH-resistant bacterium is provided. A method for detecting sensitivity to Isoniazid in *M. tuberculosis,* comprising the steps of obtaining a furA gene of *M. tuberculosis* in a sample and detecting a mutation involved in resistance to Isoniazid with respect to the obtained furA gene. The method of the invention may, if necessary, comprise a step of detecting a mutation in a fabG1 gene of *M. tuberculosis,* and may preferably be combined with another known method for detecting sensitivity to Isoniazid.

SEQUENCE LISTING

<110>  Nipro Corporation
       JAPAN as represented by President of International Medical Center of Japa

<120>  Method and strip for detection of isoniazid-sensitive mycobacterium

<130>  P209N21059

<150>  JP 2008-173477
<151>  2008-07-02

<160>  18

<170>  PatentIn version 3.1

<210>  1
<211>  444
<212>  DNA
<213>  Mycobacterium tuberculosis

<220>
<221>  CDS
<222>  (1)..(444)
<223>

<400>  1
gtg tcc tct ata ccg gac tac gcc gaa cag ctc cgg acg gcc gac ctg       48
Val Ser Ser Ile Pro Asp Tyr Ala Glu Gln Leu Arg Thr Ala Asp Leu
1               5                   10                  15

cgc gtg acc cga ccg cgc gtc gcc gtc ctg gaa gca gtg aat gcg cat       96
Arg Val Thr Arg Pro Arg Val Ala Val Leu Glu Ala Val Asn Ala His
            20                  25                  30

cca cac gcc gac acg gaa acg att ttc ggt gcc gtg cgt ttt gcg ctg      144
Pro His Ala Asp Thr Glu Thr Ile Phe Gly Ala Val Arg Phe Ala Leu
        35                  40                  45

ccc gac gta tcc cgg caa gcc gtg tac gac gtg ctg cat gcc ctg acc      192
Pro Asp Val Ser Arg Gln Ala Val Tyr Asp Val Leu His Ala Leu Thr
    50                  55                  60

gcc gcg ggc ttg gtg cga aag atc caa ccc tcg ggc tcc gtc gcg cgc      240
Ala Ala Gly Leu Val Arg Lys Ile Gln Pro Ser Gly Ser Val Ala Arg
65                  70                  75                  80

tac gag tcc agg gtc ggc gac aac cac cat cac atc gtc tgc cgg tct      288
Tyr Glu Ser Arg Val Gly Asp Asn His His His Ile Val Cys Arg Ser
                85                  90                  95

tgc ggg gtt atc gcc gat gtc gac tgt gct gtt ggc gag gca ccc tgt      336
Cys Gly Val Ile Ala Asp Val Asp Cys Ala Val Gly Glu Ala Pro Cys
            100                 105                 110

ctg acg gcc tcg gac cat aac ggc ttc ctg ttg gac gag gcg gag gtc      384
Leu Thr Ala Ser Asp His Asn Gly Phe Leu Leu Asp Glu Ala Glu Val
        115                 120                 125

atc tac tgg ggt cta tgt cct gat tgt tcg ata tcc gac act tcg cga      432
Ile Tyr Trp Gly Leu Cys Pro Asp Cys Ser Ile Ser Asp Thr Ser Arg

130                 135                 140

tca cat ccg tga                                                   444
Ser His Pro
145


<210>  2
<211>  147
<212>  PRT
<213>  Mycobacterium tuberculosis

<400>  2

Val Ser Ser Ile Pro Asp Tyr Ala Glu Gln Leu Arg Thr Ala Asp Leu
1               5                   10                  15


Arg Val Thr Arg Pro Arg Val Ala Val Leu Glu Ala Val Asn Ala His
            20                  25                  30


Pro His Ala Asp Thr Glu Thr Ile Phe Gly Ala Val Arg Phe Ala Leu
        35                  40                  45


Pro Asp Val Ser Arg Gln Ala Val Tyr Asp Val Leu His Ala Leu Thr
    50                  55                  60


Ala Ala Gly Leu Val Arg Lys Ile Gln Pro Ser Gly Ser Val Ala Arg
65                  70                  75                  80


Tyr Glu Ser Arg Val Gly Asp Asn His His His Ile Val Cys Arg Ser
                85                  90                  95


Cys Gly Val Ile Ala Asp Val Asp Cys Ala Val Gly Glu Ala Pro Cys
            100                 105                 110


Leu Thr Ala Ser Asp His Asn Gly Phe Leu Leu Asp Glu Ala Glu Val
        115                 120                 125


Ile Tyr Trp Gly Leu Cys Pro Asp Cys Ser Ile Ser Asp Thr Ser Arg
    130                 135                 140


Ser His Pro
145


<210>  3
<211>  21
<212>  DNA
<213>  Artificial

<220>
<223>  Designed DNA based on furA gene (probe)

<400>  3
gctccggacg gccgacctgc g                                                21

<210>  4
<211>  25
<212>  DNA
<213>  Artificial

<220>
<223>  Designed DNA based on furA gene (primer)

<400>  4
ggctcatcgg aacatacgaa ggctg                                            25

<210>  5
<211>  19
<212>  DNA
<213>  Artificial

<220>
<223>  Designed DNA based on furA gene (primer)

<400>  5
gtcgtacacg gcttgccgg                                                   19

<210>  6
<211>  20
<212>  DNA
<213>  Artificial

<220>
<223>  Designed DNA based on furA gene (primer)

<400>  6
gccatcccac gatccagcgg                                                  20

<210>  7
<211>  21
<212>  DNA
<213>  Artificial

<220>
<223>  Designed DNA based on furA gene (primer)

<400>  7
gtcgggcagc gcaaaacgca c                                                21

<210>  8
<211>  744
<212>  DNA
<213>  Mycobacterium tuberculosis

<220>
<221>  CDS
<222>  (1)..(744)

<223>

<400> 8
gtg act gcc aca gcc act gaa ggg gcc aaa ccc cca ttc gta tcc cgt      48
Val Thr Ala Thr Ala Thr Glu Gly Ala Lys Pro Pro Phe Val Ser Arg
1               5                   10                  15

tca gtc ctg gtt acc gga gga aac cgg ggg atc ggg ctg gcg atc gca      96
Ser Val Leu Val Thr Gly Gly Asn Arg Gly Ile Gly Leu Ala Ile Ala
            20                  25                  30

cag cgg ctg gct gcc gac ggc cac aag gtg gcc gtc acc cac cgt gga      144
Gln Arg Leu Ala Ala Asp Gly His Lys Val Ala Val Thr His Arg Gly
        35                  40                  45

tcc gga gcg cca aag ggg ctg ttt ggc gtc gaa tgt gac gtc acc gac      192
Ser Gly Ala Pro Lys Gly Leu Phe Gly Val Glu Cys Asp Val Thr Asp
    50                  55                  60

agc gac gcc gtc gat cgc gcc ttc acg gcg gta gaa gag cac cag ggt      240
Ser Asp Ala Val Asp Arg Ala Phe Thr Ala Val Glu Glu His Gln Gly
65                  70                  75                  80

ccg gtc gag gtg ctg gtg tcc aac gcc ggc cta tcc gcg gac gca ttc      288
Pro Val Glu Val Leu Val Ser Asn Ala Gly Leu Ser Ala Asp Ala Phe
                85                  90                  95

ctc atg cgg atg acc gag gaa aag ttc gag aag gtc atc aac gcc aac      336
Leu Met Arg Met Thr Glu Glu Lys Phe Glu Lys Val Ile Asn Ala Asn
            100                 105                 110

ctc acc ggg gcg ttc cgg gtg gct caa cgg gca tcg cgc agc atg cag      384
Leu Thr Gly Ala Phe Arg Val Ala Gln Arg Ala Ser Arg Ser Met Gln
        115                 120                 125

cgc aac aaa ttc ggt cga atg ata ttc ata ggt tcg gtc tcc ggc agc      432
Arg Asn Lys Phe Gly Arg Met Ile Phe Ile Gly Ser Val Ser Gly Ser
    130                 135                 140

tgg ggc atc ggc aac cag gcc aac tac gca gcc tcc aag gcc gga gtg      480
Trp Gly Ile Gly Asn Gln Ala Asn Tyr Ala Ala Ser Lys Ala Gly Val
145                 150                 155                 160

att ggc atg gcc cgc tcg atc gcc cgc gag ctg tcg aag gca aac gtg      528
Ile Gly Met Ala Arg Ser Ile Ala Arg Glu Leu Ser Lys Ala Asn Val
                165                 170                 175

acc gcg aat gtg gtg gcc ccg ggc tac atc gac acc gat atg acc cgc      576
Thr Ala Asn Val Val Ala Pro Gly Tyr Ile Asp Thr Asp Met Thr Arg
            180                 185                 190

gcg ctg gat gag cgg att cag cag ggg gcg ctg caa ttt atc cca gcg      624
Ala Leu Asp Glu Arg Ile Gln Gln Gly Ala Leu Gln Phe Ile Pro Ala
        195                 200                 205

aag cgg gtc ggc acc ccc gcc gag gtc gcc ggg gtg gtc agc ttc ctg      672
Lys Arg Val Gly Thr Pro Ala Glu Val Ala Gly Val Val Ser Phe Leu
    210                 215                 220

gct tcc gag gat gcg agc tat atc tcc ggt gcg gtc atc ccg gtc gac      720
Ala Ser Glu Asp Ala Ser Tyr Ile Ser Gly Ala Val Ile Pro Val Asp

225                 230                 235                 240

ggc ggc atg ggt atg ggc cac tga                                   744
Gly Gly Met Gly Met Gly His
                245

<210>  9
<211>  247
<212>  PRT
<213>  Mycobacterium tuberculosis

<400>  9

Val Thr Ala Thr Ala Thr Glu Gly Ala Lys Pro Pro Phe Val Ser Arg
1               5                   10                  15

Ser Val Leu Val Thr Gly Gly Asn Arg Gly Ile Gly Leu Ala Ile Ala
            20                  25                  30

Gln Arg Leu Ala Ala Asp Gly His Lys Val Ala Val Thr His Arg Gly
        35                  40                  45

Ser Gly Ala Pro Lys Gly Leu Phe Gly Val Glu Cys Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Ser
    130                 135                 140

Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190

Ala Leu Asp Glu Arg Ile Gln Gln Gly Ala Leu Gln Phe Ile Pro Ala
        195                 200                 205

Lys Arg Val Gly Thr Pro Ala Glu Val Ala Gly Val Val Ser Phe Leu
    210                 215                 220

Ala Ser Glu Asp Ala Ser Tyr Ile Ser Gly Ala Val Ile Pro Val Asp
225                 230                 235                 240

Gly Gly Met Gly Met Gly His
                245

<210>  10
<211>  20
<212>  DNA
<213>  Artificial

<220>
<223>  Designed DNA based on fabG1 gene (probe)

<400>  10
ggggcgctgc aatttatccc                                                   20

<210>  11
<211>  22
<212>  DNA
<213>  Artificial

<220>
<223>  Designed DNA based on fabG1 gene (primer)

<400>  11
gtcgaaggca aacgtgaccg cg                                                22

<210>  12
<211>  21
<212>  DNA
<213>  Artificial

<220>
<223>  Designed DNA based on fabG1 gene (primer)

<400>  12
gtccagcagt cctgtcatgt g                                                 21

<210>  13
<211>  24
<212>  DNA
<213>  Artificial

<220>
<223>  Designed DNA based on fabG1 gene (primer)

<400>  13
ggctacatcg acaccgatat gacc                                              24


<210>  14
<211>  23
<212>  DNA
<213>  Artificial

<220>
<223>  Designed DNA based on fabG1 gene (primer)

<400>  14
gcgtccttgt gttgtgtcag tgg                                               23


<210>  15
<211>  20
<212>  DNA
<213>  Artificial

<220>
<223>  Designed DNA based on katG gene (primer)

<400>  15
gctcatcgga acatacgaag                                                   20


<210>  16
<211>  30
<212>  DNA
<213>  Artificial

<220>
<223>  Designed DNA based on katG gene (primer)

<400>  16
gtgctgcggc gggttgtggt tgatcggcgg                                        30


<210>  17
<211>  20
<212>  DNA
<213>  Artificial

<220>
<223>  Designed DNA based on inhA gene (primer)

<400>  17
ttcgtagggc gtcaatacac                                                   20


<210>  18
<211>  20
<212>  DNA
<213>  Artificial

<220>
<223>  Designed DNA based on inhA gene (primer)

<400>  18

ccgaacgaca gcagcaggac 20

## Description

### Technical Field

The present invention relates to a method and test strip for detecting sensitivity to Isoniazid in *Mycobacterium tuberculosis.*

### Background Art

Currently, it is said that tuberculosis kills several thousand people in Japan each year and several million people on a global scale each year. Treatment of tuberculosis is basically performed with medical treatment including primarily chemotherapy, and when medical treatment cannot achieve the goal of the treatment, surgical treatment is taken into account.

A wide variety of drugs such as Isoniazid (INH), rifampicin (RFP), streptomycin (SM), ethambutol (EB), and pyrazinamide (PZA) are known as drugs for use in medical treatment for tuberculosis. However, it is the problem that administration of a drug to a patient results in the occurrence of a resistant bacterium that has acquired resistance to the administered drug by mutation.

Drug-resistant bacteria are reported to acquire drug resistance as a result of mutation in the specific gene. Therefore, methods for detecting a drug-resistant bacterium by detecting a mutation in the specific gene have been developed. For example, a kit for diagnosing *M. tuberculosis* is disclosed which contains a basal plate on which oligonucleotides synthesized based on base sequences of genes, which are associated with resistance to a drug for the treatment of tuberculosis and are on the *M. tuberculosis* genome of a wild-type *M. tuberculosis* sensitive to a drug for the treatment of tuberculosis and of a mutant *M. tuberculosis* resistant to any drug, are immobilized (Patent Document 1). A currently developed DNA microarray kit "Oligo Array" (Nisshinbo Industries, Inc.) is a kit capable of detecting gene mutations involved in resistance to five drugs, namely, INH, RFP, SM, kanamycin (KM), and EB.

Isoniazid (INH) is the most representative drug used in the treatment of tuberculosis. Many of INH-resistant bacteria have mutations in a katG gene or an inhA gene. Various methods for detecting these mutations are being investigated (Patent Documents 2 and 3, for example). However, even though about 80% of the INH-resistant bacteria have known mutations in the katG gene or the inhA gene and thus can be detected as resistant bacteria, the other about 20% of the INH-resistant bacteria cannot be detected. Accordingly, INH is administered even to tuberculosis patients carrying such undetectable INH-resistant bacteria.

There is a report that the above-described katG gene encoding catalase-peroxidase constitutes a furA-katG operon starting with a furA gene, and the furA gene is a negative transcription gene of this operon (Non-Patent Document 1). The furA gene regulates katG expression and is therefore involved in the INH resistance. Non-Patent Document 1 only confirms the function of the furA gene by deleting the entire furA gene but does not describe mutations in the furA gene at all.

### Prior Art Documents

Patent document 1: Japanese Laid-Open Patent Publication No. 2001-103981
Patent document 2: Japanese Patent Publication No. 3579049
Patent document 3: Japanese National Publication No. 9-501823

Non-Patent document 1: A.S. Pym et al., Molecular Microbiology, 2001, 40, 879-889
Non-Patent document 2: Parish et al., J. Bact., 2007, 189(10), 3721-3728
Non-Patent document 3: Cohen-Gonsaud et al., J. Mol. Biol., 2002, 320(2), 249-261
Non-Patent document 4: Marrakchi et al., Microbiology, 2002, 148(Pt4), 951-960

### Summary of the Invention

### Problems to be Solved by the Invention

It is an object of the present invention to provide novel means for more reliably detecting sensitivity to INH in *M. tuberculosis,* that is, detecting whether or not *M. tuberculosis* is an INH-resistant bacterium.

### Means for Solving the Problems

The inventors of the present invention found that the furA gene is also one of Isoniazid-sensitivity genes and an INH-resistant bacterium can be identified based on the presence or absence of a mutation in the furA gene.

The invention provides a method for detecting sensitivity to Isoniazid in *M. tuberculosis,* comprising the steps of:
obtaining a furA gene of *M. tuberculosis* in a sample; and
detecting a mutation involved in resistance to Isoniazid with respect to the obtained furA gene.

In one embodiment, the above mutation is a mutation of a base from C to T at the 41st position in a base sequence shown in SEQ ID NO: 1 of the Sequence Listing or a mutation in its complementary sequence.

The invention further provides a test strip for detecting sensitivity to Isoniazid in *M. tuberculosis,*
wherein at least one probe is immobilized on the test strip, and
wherein the at least one probe is constituted by an oligonucleotide that is capable of hybridizing to a region of a base sequence having a mutation in a furA gene of *M. tuberculosis* or constituted by an oligonucleotide that is, in the region of the base sequence having the mutation in the furA gene of the *M. tuberculosis,* incapable of hybridizing to the region of the base sequence having the mutation but capable of hybridizing to a region of a wild-type base sequence corresponding to the region.

In one embodiment, the above mutation in the furA gene is a mutation of a base from C to T at the 41st position in a base sequence shown in SEQ ID NO: 1 of the Sequence Listing or a mutation in its complementary sequence.

In a further embodiment, the above oligonucleotide that is capable of hybridizing to the region of the wild-type base sequence is constituted by a base sequence shown in SEQ ID NO: 3 of the Sequence Listing or its complementary sequence.

In one embodiment, a probe constituted by an oligonucleotide that is capable of hybridizing to a region of a base sequence having a mutation in a fabG1 gene of *M. tuberculosis* or constituted by an oligonucleotide that is, in the region of the base sequence having the mutation in the fabG1 gene of the *M. tuberculosis,* incapable of hybridizing to the region of the base sequence having the mutation but capable of hybridizing to a region of a wild-type base sequence corresponding to the region in the fabG1 gene is further immobilized on the test strip.

In one embodiment, the above mutation in the fabG1 gene is a mutation of a base from G to A or T at the 609th position in a base sequence shown in SEQ ID NO: 8 of the Sequence Listing or a mutation in its complementary sequence.

In a further embodiment, the above oligonucleotide that is capable of hybridizing to the region of the wild-type base sequence of the fabG1 gene is constituted by a base sequence shown in SEQ ID NO: 10 of the Sequence Listing or its complementary sequence.

The invention further provides a kit for detecting sensitivity to Isoniazid in *M. tuberculosis,* comprising the test strip of any of the above.

The invention further provides a method for detecting sensitivity to Isoniazid in *M. tuberculosis,* comprising the steps of:
obtaining a furA gene of *M. tuberculosis* in a sample;
bringing the obtained furA gene into contact with the test strip of any of the above to bind the furA gene to the probe immobilized on the test strip; and
allowing color development of the furA gene bound to the probe.

In one embodiment, the above method further comprising the steps of:
obtaining a fabG1 gene of *M. tuberculosis* in the sample:
   bringing the obtained fabG1 gene into contact with the test strip of any of the above to bind the fabG1 gene to the probe immobilized on the test strip; and
   allowing color development of the fabG1 gene bound to the probe.

### Effect of the Invention

According to the present invention, some INH-resistant bacteria that conventionally have not been identified as INH-resistant bacteria can also be accurately identified as INH-resistant bacteria. Therefore, by combining the invention with an existing method for detecting a mutation in the katG gene or the inhA gene, about 90% of the INH-resistant bacteria can be identified as INH-resistant bacteria. In this manner, the INH sensitivity can be detected more reliably. It is consequently possible to provide appropriate treatment to a tuberculosis patient carrying an Isoniazid-resistant *M. tuberculosis.*

### Mode for Carrying Out the Invention

### (Mutation in furA Gene and fabG1 Gene)

In the present invention, an Isoniazid (INH)-sensitivity gene of *M. tuberculosis* refers to a gene associated with the effect of Isoniazid (INH), which is a drug for the treatment of tuberculosis. An INH-resistant bacterium has undergone a mutation in this wild-type INH-sensitivity gene and has acquired drug resistance with respect to the effect of INH. Specifically, in an enzyme expressed from an INH-sensitivity gene having a mutation, substitution, insertion, and/or deletion has occurred in a sequence of amino acids constituting the enzyme, and thus a reduction in enzyme activity or a structural change at a drug-binding site occurs, thereby inhibiting the effect of INH.

The resistance to INH in *M. tuberculosis* may be caused by mutations in various genes associated with the effect of INH, such as the katG gene and the inhA gene, as described above. Such mutations are disclosed in Patent Documents 1 to 3, for example. A *M. tuberculosis* having any one of these mutations can be INH-resistant.

In the invention, it was newly found that besides the above-described genes, a mutation in the furA gene may also change *M. tuberculosis* to an INH-resistant bacterium. As described above, the furA gene is the starting gene in the furA-katG operon and is the negative transcription gene of that operon (Non-Patent Document 1).

Specifically, the mutation in the furA gene includes a mutation of a base from C to T at the 41st position in a base sequence shown in SEQ ID NO: 1 of the Sequence Listing and a mutation of a base from G to A in its complementary sequence. In the invention, this mutation will be collectively described as "a mutation of a base from C to T at the 41st position in a base sequence or a mutation in its complementary sequence". This mutation causes a mutation from alanine (Ala) to valine (Val) at the 14th position in FurA (SEQ ID NO: 2 of the Sequence Listing) encoded by the furA gene.

In the invention, it also was newly found that a fabG1 gene may have a mutation and this mutation may change *M. tuberculosis* to an INH-resistant bacterium. The fabG1 gene is the starting gene in a fabG1-inhA operon and is a gene encoding a mycolic acid-synthesizing enzyme (Non-Patent Documents 2 to 4).

Specifically, the mutation in the fabG1 gene includes a mutation of a base from G to A or T at the 609th position in a base sequence shown in SEQ ID NO: 8 of the Sequence Listing and a mutation of a base from C to T or A in its complementary sequence. In the invention, this mutation will be collectively described as "a mutation of a base from G to A or T at the 609th position in a base sequence or a mutation in its complementary sequence". This mutation is a silent mutation of leucine (Leu) at the 203rd position in FabG1 (SEQ ID NO: 9 of the Sequence Listing) encoded by the fabG1 gene.

Usually used means described in, for example, Nollau et al., Clin. Chem., 43, 1114-1120 (1997); "Laboratory Protocols for Mutation Detection" (Landegren, U. et al., Oxford University Press (1996)); and "PCR", 2nd Edition (Newton et al., BIOS Scientific Publishers Limited (1997)) can be applied as the method for detecting the above-described mutations on the furA gene and the fabG1 gene. Specifically, the detection method can be selected from known methods including, but not limited to, methods such as a PCR fragment sequencing method; a single-stranded conformational polymorphism method (an SSCP method: Orita, M. et al., Proc. Natl. Acad. Sci. USA., 86(8), 2766-2770 (1989)); a heteroduplex denaturing gradient gel electrophoresis method (a DGGE method: Sheffield, V. C. et al., Proc. Natl. Acad. Sci. USA., 86(1), 232-236 (1989)); an Invader method (Griffin, T. J. et al., Trend Biotech, 18, 77 (2000)); a SniPer ™ method (Amersham Pharmacia Biotech); a TaqMan PCR method (Livak, K. J., Genel. Anal., 14, 143 (1999); Morris, T. et al., J. Clin. Microbiol., 34, 2933(1996)); a MALDI-TOF/MS method (Griffin, T. J. et al., Proc. Natl. Acad. Sci. USA., 96(11), 6301-6 (1999)); a restriction fragment length polymorphism analysis method (RFLP: Murray, J. C. et al, Proc. Natl. Acad. Sci. USA., 80(19), 5951-5955 (1983)); a DNA chip hybridization method (Kokoris, K. et al., J. Med. Genet., 36, 730 (1999)); and a Masscode ™ method (Qiagen Genomics). Any means can be applied as long as it is a method for detecting a gene mutation. For example, a PCR-SSOP (sequence-specific oligonucleotide probes) method can also be used. The PCR-SSOP method is a method, in which a probe that is about 10 to about 30 bases long containing a mutation site and is perfectly complementary to one of allele sequences is produced, and after amplification of DNA containing the mutation site by a PCR method, hybridization is performed, with the result that gene polymorphism is determined based on the presence or absence of hybrid formation.

### (Probe)

Probes of the invention used to detect sensitivity to Isoniazid in *M. tuberculosis* include an oligonucleotide probe that is capable of binding (hybridizing) to a region having a mutation in a furA gene of *M. tuberculosis* (hereinafter sometimes referred to as "furA mutated probe") and an oligonucleotide probe that is, in this region having the mutation in the furA gene, incapable of hybridizing to the region of the base sequence having the mutation but capable of hybridizing to a region of a wild-type base sequence corresponding to that region (hereinafter sometimes referred to as "furA wild-type probe").

Furthermore, probes include an oligonucleotide probe that is capable of binding (hybridizing) to a region having a mutation in the fabG1 gene of *M. tuberculosis* (hereinafter sometimes referred to as "fabG1 mutated probe") and an oligonucleotide probe that is, in this region having the mutation in the fabG1 gene, incapable of hybridizing to the region of the base sequence having the mutation but capable of hybridizing to a region of a wild-type base sequence corresponding to that region (hereinafter sometimes referred to as "fabG1 wild-type probe").

The length of each probe depends on the temperature during binding (hybridization), but is generally 10 to 30 nucleotides and preferably 12 to 26 nucleotides.

In the invention, a mutated probe can bind (hybridize) to a region having the above-described mutation in the furA gene or the fabG1 gene. The sequence of bases other than the mutated base in a region having such a mutation is wild-type. When this region is wild-type, the mutated probe cannot bind to this region. Moreover, in order to reliably detect a mutation, it is preferable to design a mutated probe so that the position of the mutation is located in the middle of an oligonucleotide constituting the mutated probe.

In the invention, a wild-type probe may bind (hybridize) to any region of a wild-type furA gene or fabG1 gene corresponding to a region having the above-described mutation.

The above-described probes can be obtained by using a standard program and primer analysis software, for example, Primer Express (PerkinElmer), and can be synthesized by using standard means such as an automated oligonucleotide synthesizer.

Furthermore, each probe may be modified at either the 5' end or the 3' end, for example, in order to immobilize the probe on a test strip.

Each probe may be provided in any form as long as the probe enables detection, and may be in the form of a solution or may be immobilized on a carrier. In order to facilitate detection, the probe is preferably immobilized on a test strip.

### (Test Strip)

On a test strip of the invention for detecting sensitivity to Isoniazid in *M. tuberculosis* at least one probe is immobilized. The at least one immobilized probe is the above-described furA mutated probe or furA wild-type probe. Preferably, the furA mutated probe and the furA wild-type probe are immobilized at mutually different positions on the test strip. When the furA gene has a mutation, the furA gene is bound to the furA mutated probe, but is not bound to the furA wild-type probe. On the other hand, when the furA gene has no mutation, the furA gene is bound to the furA wild-type probe. Therefore, when *M. tuberculosis* is sensitive to INH, the furA gene is bound only to the furA wild-type probe, and when *M. tuberculosis* is resistant to INH, the furA gene is bound to the furA mutated probe without being bound to the furA wild-type probe.

Furthermore, on the test strip of the invention, a control or marker for confirming color development may be immobilized.

Furthermore, on the test strip of the invention for detecting sensitivity to Isoniazid in *M. tuberculosis,* the above-described fabG1 mutated probe or fabG1 wild-type probe may be immobilized. More preferably, the fabG1 mutated probe and fabG1 wild-type probe are immobilized at mutually different positions on the test strip. When the fabG1 gene has a mutation, the fabG1 gene is bound to the fabG1 mutated probe, but is not bound to the fabG1 wild-type probe. On the other hand, when the fabG1 gene has no mutation, the fabG1 gene is bound to the fabG1 wild-type probe. Therefore, when *M. tuberculosis* is sensitive to INH, the fabG1 gene is bound only to the fabG1 wild-type probe, and when *M. tuberculosis* is resistant to INH, the fabG1 gene is bound to the fabG1 mutated probe without being bound to the fabG1 wild-type probe.

Furthermore, on the test strip of the invention, a known probe that can detect INH sensitivity or INH resistance other than those with respect to the furA gene and the fabG1 gene may also be immobilized.

The test strip of the invention can be created by physically or chemically immobilizing the above-described probe onto the surface of a carrier. Examples of the carrier include, but not particularly limited to, organic materials such as vinyl polymer, nylon, polyester, polyethylene, polypropylene, polystyrene, polyethylene terephthalate, and nitrocellulose; inorganic materials such as glass and silica; and metallic materials such as gold and silver. Organic materials are preferable, and polyesters such as polyethylene terephthalate are more preferable, because of their easily moldable properties. Preferably, these carriers are white so that it is easy to visually confirm color development in performing detection by a color development method.

Preferably, the above-described different types of probes are independently disposed on the above-described carrier at a constant spacing. Various known methods can be used as the method for physically immobilizing a probe onto the surface of the carrier. An example thereof is a method for coating the surface of a carrier with a polycationic polymer such as polylysine. With this method, the immobilization efficiency can be increased by electrostatic interaction with the probe, which is a polyanion. Another example is a method for adding an irrelevant base sequence (e.g., a polythymine chain) to an end of the probe to increase the molecular weight of the actual probe to be immobilized, thereby increasing the immobilization efficiency. For example, various probes can be immobilized relatively easily by discharging a solution containing a polythymine-added oligonucleotide probe from a dispenser onto a nitrocellulose membrane, and then irradiating the membrane with ultraviolet rays. Specifically, when probes are placed in dispensers each equipped with a 24-gauge needle and applied from the dispensers onto a nitrocellulose membrane at a constant spacing between the probes by discharging the probes at a rate of 0.5 to 1.0 µL/minute while moving the dispensers at an application speed of 2.5 to 8.5 mm/second, the probes are applied as about 1 to 2 mm wide stripes extending side by side at the constant spacing. The carrier on which the stripes of the probes have been applied is irradiated with ultraviolet rays, and as a result, the probes are immobilized onto the carrier. Furthermore, the carrier is cut into thin pieces across these stripes, if necessary, and thus, multiple test strips on which the probes are disposed in order can be obtained all at once.

It is also known that in a case where the surface of the carrier is a metallic material such as gold, treatment of the surface of the carrier with a thiol having an amino group, such as 2-aminoethanethiol, or disulfide compound improves the immobilization efficiency through electrostatic interaction with the probe, which is a polyanion.

Various known methods can be used as the method for chemically immobilizing a probe by introducing a functional group into the probe. For example, in a case where the material for the carrier is an inorganic material such as glass or silicon, a method for modifying an end of the probe with a functional group that can cause a silane coupling reaction, such as a trimethoxysilyl group or a triethoxysilyl group, can be used, where a test strip can be obtained by soaking the carrier in a solution containing the thus modified probe for 24 to 48 hours and washing the carrier after the removal from the solution. Alternatively, a method for immobilizing a probe by treating a carrier made of an inorganic material such as glass or silicon with a silane coupling agent having an amino group such as aminoethoxysilane so as to aminate the surface of the carrier, and then causing the carrier to undergo an amino coupling reaction with the probe having a carboxylic acid introduced at its end can be used. Furthermore, in a case where the material for the carrier is a metallic material such as gold or silver, immobilization can be achieved by modifying an end of the probe with a functional group that is capable of binding to a metal, such as a thiol group or a disulfide group, soaking the carrier in a solution containing this modified probe for 24 to 48 hours, and washing the carrier after the removal from the solution.

Moreover, instead of the method for immobilizing a synthesized probe, a method for directly synthesizing a probe having a desired sequence on the surface of a carrier by means of lithography also is known.

### (Method for Detecting Sensitivity to INH Using Test Strip)

### 1. Sample

In the invention, a "sample" usually is any sample that is necessary for a test for *M. tuberculosis.* Examples of the sample include body fluids such as sputum, pharyngeal swab, gastric juice, bronchoalveolar lavage fluid, endotracheal aspirate, throat swab and/or a tissue biopsy sample, and cultures obtained by culturing these body fluids.

### 2. Pretreatment of Sample (DNA Extraction)

Extraction of DNA from the above-described sample can be performed by a known method. Examples of such method include a phenol extraction method, a guanidine thiocyanate extraction method, and a vanadyl ribonucleoside complex extraction method.

### 3. Amplification of Nucleic Acid

A target gene is obtained from the extracted DNA. In a case where the target gene is amplified by PCR, amplification is performed using, for example, the following steps: (1) a denaturation step of converting a double-stranded genome DNA to single strands by heat treatment under reaction conditions at about 92 to 95°C for about 30 seconds to 1 minute; (2) an annealing step of producing a double-stranded portion that serves as a starting point of the PCR reaction by binding at least two types of amplification primers to each of the single-stranded DNAs under reaction conditions at about 50 to 65°C for about 20 seconds to 1 minute; (3) a chain extension step of causing a reaction using a DNA polymerase under reaction conditions at about 70 to 75°C for about 20 seconds to 5 minutes; and a step of repeating the steps (1) to (3) 20 to 40 times by a usual method.

The primer pair used to amplify the furA gene by PCR includes oligonucleotides having the same or complementary base sequences that contain a sequence upstream of a sequence site containing a mutation site and a sequence downstream of that site in order to amplify the furA gene containing the mutation site. A preferred example of the primer pair is oligonucleotides of SEQ ID NO: 6 (gccatcccac gatccagcgg) and SEQ ID NO: 7 (gtcgggcagc gcaaaacgca c) in the Sequence Listing or oligonucleotides having sequences complementary to these sequences. On the other hand, the primer pair used to amplify the fabG1 gene by PCR includes oligonucleotides having the same or complementary base sequences that contain a sequence upstream of a sequence site containing a mutation site and a sequence downstream of that site in order to amplify the fabG1 gene containing the mutation site. A preferred example of the primer pair is oligonucleotides having sequences of SEQ ID NO: 13 (ggctacatcg acaccgatat gacc) and SEQ ID NO: 14 (gcgtccttgt gttgtgtcag tgg) in the Sequence Listing or oligonucleotides having sequences complementary to these sequences.

Moreover, in order to improve the measurement sensitivity, for example, in case of directly amplifying DNA from a sample, nucleic acid amplification before the above-described PCR reaction may be further performed by a Nested PCR method (Japanese Patent Publication No. 6-81600B) or the like. Sequences outside of the sequences of the primers used in the above-described PCR reaction can be selected for primers used in the Nested PCR method. For example, in case of the furA gene, oligonucleotides of SEQ ID NO: 4 (ggctcatcgg aacatacgaa ggctg) and SEQ ID NO: 5 (gtcgtacacg gcttgccgg) in the Sequence Listing or oligonucleotides having sequences complementary to these sequences can be used, and in case of the fabG1 gene, oligonucleotides of SEQ ID NO: 11 (gtcgaaggca aacgtgaccg cg) and SEQ ID NO: 12 (gtccagcagt cctgtcatgt g) in the Sequence Listing or oligonucleotides having sequences complementary to these sequences can be used.

The sequences of the above-described probes and primers can be obtained by using a standard program and primer analysis software, for example, Primer Express (manufactured by PerkinElmer), and can be synthesized by using standard means such as an automated oligonucleotide synthesizer.

### 4. Method for Detecting Sensitivity to INH in M. tuberculosis

In the invention, the sensitivity to INH in *M. tuberculosis* can be detected by the PCR-SSOP method. More conveniently, the INH sensitivity can also be detected by comparing the positions of immobilized probes with the position of a spot detected by the PCR-SSOP method. For example, in a case where a test strip on which the above-described probes are immobilized is used, it is preferable to perform hybridization of the PCR-SSOP method at a temperature of about 60 to 65°C in that a nonspecific binding reaction is unlikely to occur. It can be determined that when the position of the detected spot is the same as the position of the furA wild-type probe, tuberculosis bacillus has no mutation, that is, tuberculosis bacillus is sensitive to INH, whereas when the position of the detected spot is the same as the position of the furA mutated probe, *M. tuberculosis* is resistant to INH. Moreover, when the position of the detected spot is the same as the position of the fabG1 wild-type probe, *M. tuberculosis* is determined to be sensitive to INH, whereas when the position of the detected spot is the same as the position of the fabG1 mutated probe, *M. tuberculosis* can be determined to be resistant to INH.

In order to easily detect the detected spot in the PCR-SSOP method, it is preferable to amplify a gene using a primer pair modified with a labeling substance such as a radioisotope, a fluorescent substance, or a chemiluminescent substance. Among detection methods that use the above-described labeling substance, a nitro blue tetrazolium (NBT)/5-bromo-4-chloro-3-indolyl phosphatase p-toluidine salt (BCIP) color development method is preferable in that this method can be performed relatively inexpensively and easily. Specifically, detection is performed in the following manner. First, a gene having biotin at an end is amplified using a primer pair obtained by modifying the 3' end or the 5' end of the above-described primer with biotin. Then, the amplified gene is bound to a probe immobilized on a test strip by hybridization between them, and furthermore, the amplified gene bound to the probe is brought into contact with alkali phosphatase-labeled streptavidin, thereby binding the alkali phosphatase-labeled streptavidin to biotin that is present at the end of the gene. Furthermore, NBT/BCIP is added so as to react with alkali phosphatase, thereby allowing color development at the position in which the alkali phosphatase bound to the probe is present. This color development can be visually confirmed.

### (Detection Kit Containing Test Strip)

A detection kit of the invention contains the above-described test strip. Preferably, the detection kit may contain any reagents suitable for detection of the sensitivity to Isoniazid in *M. tuberculosis.* Examples of the reagents include reagents for DNA extraction as described above, a reagent for gene amplification, and a reagent for detecting the binding of a gene to a probe. A kit for performing the above-described PCR-SSOP method will be described as the specific example of the kit.

The reagents that may be contained in the kit of the invention and that are used to extract DNA and to acquire a target gene are reagents used in any method described above.

The primer pair for amplifying the furA gene by PCR may also be contained in the kit of the invention. The primer pair is oligonucleotides having the same or complementary base sequences that contain a sequence upstream of a sequence site containing a mutation site and a sequence downstream of that site in order to amplify the furA gene containing any mutation site. In order to detect the INH sensitivity, a preferred example of the primer pair for furA gene amplification is the above-described oligonucleotides of SEQ ID NOS: 6 and 7 in the Sequence Listing or oligonucleotides having sequences complementary to these sequences. On the other hand, a preferred example of the primer pair for the fabG1 gene is the above-described oligonucleotides of SEQ ID NOS: 13 and 14 in the Sequence Listing or oligonucleotides having sequences complementary to these sequences. For the primer pair used in the Nested PCR method for improving the measurement sensitivity, for example, the above-described oligonucleotides of SEQ ID NOS: 4 and 5 in the Sequence Listing or oligonucleotides having sequences complementary to these sequences can be used with respect to the furA gene, whereas the above-described oligonucleotides of SEQ ID NOS: 11 and 12 in the Sequence Listing or oligonucleotides having sequences complementary to these sequences can be used with respect to the fabG1 gene.

Furthermore, it is preferable that the above-described primer pairs are modified with a radioisotope, a fluorescent substance, a chemiluminuscent substance, or the like in order to facilitate the detection in the PCR-SSOP method.

Moreover, gene amplification can be performed by known techniques such as a LAMP method and an ICAN method other than the PCR method. In the invention, any of these methods may be used, and the invention also encompasses a kit containing any reagent used in these methods.

Furthermore, the kit of the invention may contain reagents for detection. Examples of such reagents in a case where the NBT/BCIP color development method is employed include streptavidin-modified alkali phosphatase, NBT, and BCIP.

### Examples

### (Example 1: M. tuberculosis Gene Analysis of INH-Resistant M. tuberculosis)

In this example, 92 samples determined to be INH-resistant *M. tuberculosis* by confirming their growth in an INH-containing solution (MGIT 960: manufactured by Nippon Becton Dickinson Company, Ltd.) or an INH-containing agar medium (Ogawa medium, 7H10) were obtained, and genome DNAs were extracted and purified from these 92 samples by phenol extraction.

### 1. Analysis of furA Gene

A furA gene was amplified using a primer pair shown below. The reaction conditions of PCR were as follows: denaturation at 94°C for 30 seconds; annealing at 55°C for 20 seconds; and chain extension at 72 °C for 20 seconds, and 30 cycles of these steps were performed for amplification.
furA Primer 1 gccatcccac gatccagcgg (SEQ ID NO: 6)
furA Primer 2 gtcgggcagc gcaaaacgca c (SEQ ID NO: 7)

The base sequence of the amplified furA gene in the 92 samples was examined with a sequencer. As a result, a novel mutation Ala14Val [gCc → gTc] was found in 14 samples out of the 92 samples.

### 2. Analysis of fabG1 Gene

A fabG1 gene was amplified using a primer pair shown below. The reaction conditions of PCR were as follows: denaturation at 94°C for 30 seconds; annealing at 55°C for 20 seconds; and chain extension at 72°C for 20 seconds, and 30 cycles of these steps were performed for amplification. fabG1 Primer 1 ggctacatcg acaccgatat gacc (SEQ ID NO: 13) fabG1 Primer 2 gcgtccttgt gttgtgtcag tgg (SEQ ID NO: 14)

The base sequence of the amplified fabG1 gene in the 92 samples was examined with a sequencer. As a result, a novel mutation Leu203Leu [ctG → ctA/T] was found in 15 samples out of the 92 samples. This mutation was a silent mutation in which even though there is a base change, the same amino acid is encoded.

### 3. Detection of Known Mutation

With respect to the above-described 92 samples, the katG gene and inhA gene having a known mutation were independently amplified, and the presence or absence of the known mutation was detected.

For katG, PCR was performed on a furA-katG operon using a primer that binds to 129 bases upstream from a furA start codon (gctcatcgga acatacgaag: SEQ ID NO: 15) and a primer that binds to 50 bases downstream from a katG stop codon (gtgctgcggc gggttgtggt tgatcggcgg: SEQ ID NO: 16), and the entire base sequence was determined by a DNA sequencing method. A mutation that had not been reported in previously reported articles was taken as a novel mutation.

For inhA, PCR was performed on a fabG1-inhA operon using a primer that binds to 200 bases upstream from a fabG1 start codon (ttcgtagggc gtcaatacac: SEQ ID NO: 17) and a primer that binds to 40 bases downstream from an inhA stop codon (ccgaacgaca gcagcaggac: SEQ ID NO: 18), and the entire base sequence was determined by a DNA sequencing method. A mutation that had not been reported in previously reported articles was taken as a novel mutation.

The results of the analyses described in 1 to 3 above are collectively shown in Table 1 below.

Table 1

As can be seen from Table 1, INH-resistant bacteria having known mutations were found in 67 samples out of the 92 samples, but when mutations in the furA gene and the fabG1 gene are also taken into account, resistant bacteria of 83 samples out of the 92 (90.2%) samples can be easily determined to be INH-resistant. Therefore, more reliable and convenient detection is possible.

### (Example 2: Production of Test Strip)

### 1. Design of Probe

Two probes below (probes 1 and 2: a furA wild-type probe and a fabG1 wild-type probe, respectively) were designed as probes capable of detecting the sensitivity to INH in *M. tuberculosis.* These probes are both wild-type probes that do not bind when there is a mutation but bind (hybridize) only to a wild-type gene.

Probe 1 gctccggacg gccgacctgc g (SEQ ID NO: 3)
Probe 2 ggggcgctgc aatttatccc (SEQ ID NO: 10)

### 2. Immobilization of Probe

Polythymine was added to the 5' end of each of the above-described probes 1 and 2 (SEQ ID NOS: 3 and 10, respectively) using a terminal transferase (Promega). Specifically, 0.4 µL of the terminal transferase (30 units/µL), 2 µL of thymidine triphosphate (10 pmol/µL), 2 µL of the oligonucleotide probe (50 mM), 2 µL of a reaction buffer included in the product, and 3.6 µL of purified water were mixed to prepare a reaction solution, and after reaction at 37°C for 4 hours, 90 µL of 10x SSC buffer was added to the reaction solution, thereby obtaining a 1 pmol/µL polythymine-added oligonucleotide probe.

Subsequently, the polythymine-added wild-type probes (probes 1 and 2) were independently placed in dispensers each equipped with a 24-gauge needle and applied from the dispensers onto nitrocellulose membranes (75 mm long x 150 mm wide: Whatman) in the longitudinal direction at a spacing of 2 mm so as to form 2 mm wide stripes by discharging the probes at a rate of 0.7 µL/minute while moving the dispensers at an application speed of 2.5 mm/second. Then, these nitrocellulose membranes were irradiated with ultraviolet rays at 312 nm for 2 minutes to immobilize the probes. Then, the nitrocellulose membranes were cut into pieces so that each piece included all the stripes, and thus, 5 mm x 150 mm test strips were created.

### Industrial Applicability

According to the present invention, even an INH-resistant bacterium whose INH sensitivity has conventionally been impossible to be determined can be identified. Therefore, by combining the invention with an existing method for detecting a mutation in the katG gene or inhA gene, about 90% of INH-resistant bacteria can be conveniently identified as INH-resistant bacteria without the need for culture, and can be easily diagnosed as untreatable with Isoniazid. As a result, a patient carrying such an INH-resistant bacterium can be provided with appropriate treatment. Therefore, it is possible to prevent unnecessary drug administration or examination, thereby making it possible to alleviate a burden on the patient, and furthermore to reduce wasteful medical spending.

## Claims

1. A method for detecting sensitivity to Isoniazid in *Mycobacterium tuberculosis,* comprising the steps of:
obtaining a furA gene of *M. tuberculosis* in a sample; and
detecting a mutation involved in resistance to Isoniazid with respect to the obtained furA gene.

2. The method of claim 1, wherein the mutation is a mutation of a base from C to T at the 41st position in a base sequence shown in SEQ ID NO: 1 of the Sequence Listing or a mutation in its complementary sequence.

3. A test strip for detecting sensitivity to Isoniazid in *Mycobacterium tuberculosis,*
wherein at least one probe is immobilized on the test strip, and
wherein the at least one probe is constituted by an oligonucleotide that is capable of hybridizing to a region of a base sequence having a mutation in a furA gene of *M. tuberculosis* or constituted by an oligonucleotide that is, in the region of the base sequence having the mutation in the furA gene of the *M. tuberculosis,* incapable of hybridizing to the region of the base sequence having the mutation but capable of hybridizing to a region of a wild-type base sequence corresponding to the region.

4. The test strip of claim 3, wherein the mutation in the furA gene is a mutation of a base from C to T at the 41st position in a base sequence shown in SEQ ID NO: 1 of the Sequence Listing or a mutation in its complementary sequence.

5. The test strip of claim 4, wherein the oligonucleotide that is capable of hybridizing to the region of the wild-type base sequence is constituted by a base sequence shown in SEQ ID NO: 3 of the Sequence Listing or its complementary sequence.

6. The test strip of any of claims 3 to 5, wherein a probe constituted by an oligonucleotide that is capable of hybridizing to a region of a base sequence having a mutation in a fabG1 gene of *M. tuberculosis* or constituted by an oligonucleotide that is, in the region of the base sequence having the mutation in the fabG1 gene of the *M. tuberculosis,* incapable of hybridizing to the region of the base sequence having the mutation but capable of hybridizing to a region of a wild-type base sequence corresponding to the region in the fabG1 gene is further immobilized on the test strip.

7. The test strip of claim 6, wherein the mutation in the fabG1 gene is a mutation of a base from G to A or T at the 609th position in a base sequence shown in SEQ ID NO: 8 of the Sequence Listing or a mutation in its complementary sequence.

8. The test strip of claim 7, wherein the oligonucleotide that is capable of hybridizing to the region of the wild-type base sequence of the fabG1 gene is constituted by a base sequence shown in SEQ ID NO: 10 of the Sequence Listing or its complementary sequence.

9. A kit for detecting sensitivity to Isoniazid in *Mycobacterium tuberculosis,* comprising the test strip of any of claims 3 to 8.

10. A method for detecting sensitivity to Isoniazid in *Mycobacterium tuberculosis,* comprising the steps of:
obtaining a furA gene of *M. tuberculosis* in a sample;
bringing the obtained furA gene into contact with the test strip of any of claims 3 to 8 to bind the furA gene to the probe immobilized on the test strip; and
allowing color development of the furA gene bound to the probe.

11. The method of claim 10, further comprising the steps of:
obtaining a fabG1 gene of *M. tuberculosis* in the sample:
bringing the obtained fabG1 gene into contact with the test strip of any of claims 6 to 8 to bind the fabG1 gene to the probe immobilized on the test strip; and
allowing color development of the fabG1 gene bound to the probe.
